# EUROPEAN PATENT SPECIFICATION

(11) **EP 4 599 857 B1**
(45) Date of publication and mention of the grant of the patent: **04.11.2026**
(21) Application number: 23874312.4
(22) Date of filing: 28.09.2023
(51) Int. Cl.: A61L 27/52, A61L 27/24, A61K 8/65

(54) **TRANSDERMAL PHOTOCURING FORMING HYDROGEL WITH BIOLOGICAL ACTIVITY, AND PREPARATION METHOD THEREFOR AND USE THEREOF**
TRANSDERMALES LICHTHÄRTENDES HYDROGEL MIT BIOLOGISCHER AKTIVITÄT UND HERSTELLUNGSVERFAHREN DAFÜR UND VERWENDUNG DAVON
HYDROGEL FORMÉ PAR PHOTO-DURCISSEMENT TRANSDERMIQUE PRÉSENTANT UNE ACTIVITÉ BIOLOGIQUE, SON PROCÉDÉ DE PRÉPARATION ET SON UTILISATION

(30) Priority: 08.10.2022 CN 202211224593; 03.04.2023 CN 202310346318
(43) Date of publication of application: 13.08.2025
(73) Proprietor: Shanxi Jinbo Biopharmaceutical Co., Ltd., Taiyuan, Shanxi 030032 (CN); Sichuan University, Chengdu, Sichuan 610065 (CN)
(72) Inventor: LIN, Hai, Chengdu, Sichuan 610065 (CN); QIU, He, Chengdu, Sichuan 610065 (CN); WANG, Jing, Chengdu, Sichuan 610065 (CN); WANG, Jian, Taiyuan, Shanxi 030032 (CN); XU, Yang, Chengdu, Sichuan 610065 (CN); SONG, Lu, Chengdu, Sichuan 610065 (CN); YANG, Xia, Taiyuan, Shanxi 030032 (CN); ZHANG, Xingdong, Chengdu, Sichuan 610065 (CN)
(74) Representative: Murgitroyd & Company
(86) International application number: PCT/CN2023/122683
(87) International publication number: WO 2024/074120

(56) References cited:
- CN-A- 101 790 360
- CN-A- 110 790 950
- CN-A- 111 116 736
- CN-A- 112 334 168
- CN-A- 113 827 779
- CN-A- 116 115 831
- JP-A- 2021 526 395
- KR-A- 20160 018 234
- KR-B1- 100 776 297
- US-A1- 2016 193 384
- C HUA, ZHU Y, XU W, YE S, ZHANG R, LU L, JIANG S: "Characterization by high-resolution crystal structure analysis of a triple-helix region of human collagen type III with potent cell adhesion activity.", BIOCHEMICAL AND BIOPHYSICAL RESEARCH COMMUNICATIONS, ELSEVIER, AMSTERDAM NL, vol. 508, no. 4, 22 January 2019 (2019-01-22), Amsterdam NL , pages 1018 - 1023, XP055677661, ISSN: 0006-291X, DOI: 10.1016/j.bbrc.2018.12.018
- SHUANG YOU: "Intravaginal Administration of Human Type III Collagen-Derived Biomaterial with High Cell-Adhesion Activity to Treat Vaginal Atrophy in Rats", ACS BIOMATERIALS SCIENCE & ENGINEERING, vol. 6, no. 4, 13 April 2020 (2020-04-13), pages 1977 - 1988, XP093154792, ISSN: 2373-9878, DOI: 10.1021/acsbiomaterials.9b01649

## Description

This application claims the priorities of (1) filing with China National Intellectual Property Administration on October 8, 2022 with application number 2022112245930, and (2) filing with China National Intellectual Property Administration on April 3, 2023 with application number 2023103463184.

### TECHNICAL FIELD

The present disclosure belongs to the technical field of bioengineering, in particular to a transdermal photocuring forming hydrogel with biological activity as well as a preparation method and an application thereof.

### BACKGROUND

Maxillofacial soft tissue has a special anatomical physiological structure, which plays a crucial role in aspects of mastication, pronunciation, and aesthetics, etc., in human, and is very vulnerable to trauma, infection and congenital diseases, etc. at the same time. Abnormalities and deficiencies in the morphology and capacity of maxillofacial soft tissue, which are manifested as facial depression, asymmetry, and aging, etc., are very common in clinical practice. They not only cause functional problems for patients, but also seriously affect their appearance and mental health, bringing many troubles to their lives. The existing clinical repair methods mainly rely on autologous soft tissue transplantation, which can lead to secondary traumatization and is not conducive to shaping. In recent years, there is an increasing demand for aesthetic restoration. How to repair the appearance of damaged tissues as much as possible while restoring its normal function, reconstructing the function of damaged tissues, and achieving good aesthetic restoration results, has become the focus of maxillofacial soft tissue repair.

Soft tissue filling is a major non-surgical cosmetic treatment project, which involves implanting soft tissue filling materials into the body and occupying cavities and defect sites caused by tissue damage or lesions, replacing or enhancing their original functions, so as to achieve the purposes of tissue repair, deformity correction, and rejuvenation treatment. It meets the needs of patients for minimally invasive surgery and is also one of the current trends in the development of plastic surgery clinical technology. The application of injectable soft tissue filling materials for non-invasive facial filling has become the most popular means of operation. Injection filling technology is simple to operate, and results can be visible in just a few minutes of injection. The process is painless and convenient, without effects on the patients in aspects of daily life and work, etc, leaving no traces, and thus privacy of the customers is kept safe. However, the ideal injectable soft tissue filling materials must possess the characteristics like biocompatibility, safety, ease of operation, fixity, and durability, etc., at the same time, which has always been a highly challenging topic for scholars studying and developing plastic and cosmetic products.

The clinical repair effect of soft tissue defects is closely related to the sources and properties of materials. In order to achieve functional and aesthetic reconstructions of damaged tissues, it is not only required that injectable filling materials have good biocompatibility and immediate filling effect, but also that implanted materials have good tissue repair and regeneration functions, with a wide range of sources and ease of operation at the same time. Although injectable soft tissue filling agents such as hyaluronic acid and carboxymethyl cellulose, etc. are widely developed and applied, they lack of various biological signaling molecules required for cell adhesion, proliferation, and differentiation, and their ability to resist enzymolysis and free radical degradation is limited, making them difficult to regulate cell behaviors or promote tissue repair. For example, cross-linked hyaluronic acid and its derivatives as physical volume filling agents are not conducive to cell adhesion, with limited ability to induce tissue regeneration, and high metabolic rate, which result in limited effectiveness in maintaining the injection results. At the same time, the implantation of cross-linked hyaluronic acid products relies on the surgeon's shaping during the operation, which requires a high level of anatomical theoretical knowledge and surgical skills, meanwhile translation of the filling material may happen, which seriously affects the implantation outcome. Document CN 112 334 168A discloses an injectable dermal filler comprising collagen, a photoinitiator and methacrylated hyaluronic acid.

Therefore, the current injection materials have problems such as insufficient mechanical strength, short duration of injection effect maintenance, poor biocompatibility with human tissues, and unnatural effects after injection, etc., making it difficult to meet the needs of practical applications.

### CONTENTS OF THE INVENTION

The object of the present invention is to provide a transdermal photocuring forming hydrogel with biological activity as well as a preparation method and a non therapeutic use thereof in view of the shortcomings of the present technology as defined by the claims. The said hydrogel has good biocompatibility and can be formed in situ in a transdermal photo-crosslinking manner after injection, it can release recombinant collagen with biological activity while achiveing the purpose of tissue filling, so as to promote the effect of tissue repair.

In order to achieve the above object, the present invention adopts the following technical solution:

In the first aspect, the present invention provides a transdermal photocuring forming hydrogel with biological activity as defined in claims 1-9. The synthetic raw materials of the hydrogel include an anhydride, a natural polysaccharide, an activating agent, an amino vicinal-diol and an oxidizing agent;

The amino acid sequence of the recombinant collagen comprises N basic repetitive units, and each basic repetitive unit contains n1 of the following characteristic amino acid sequences: "G-Xaa₁-Xaa₂-G-E-Xaa₃"; the 3' end and the 5' end of the basic repetitive unit are connected to form the above characteristic amino acid sequence;

Wherein, N is an integer greater than 4; and n1 is an integer greater than 3.

Further, the N value is an integer ranging from 4 to 300.

Further, the N value is an integer ranging from 4 to 200. It includes but is not limited to 4, 5, 6, 7, 8, 9, 10, 11, 12, 13, 14, 15, 16, 17, 18, 19, 20, 21, 22, 23, 24, 25, 26, 27, 28, 29, 30.......200.

Further, the valuing of the N value can enable the molecular weight of recombinant collagen to reach from 1 kDa to 200 kDa, and further from 3 kDa to 150 kDa.

Further, the characteristic amino acid sequences are arranged in the basic repetitive unit continuously or at intervals.

Further, the amino acid sequence of the recombinant collagen presents the following characteristics:

[-G-E-Xaa₃-Yaa₁-(G-Xaa₁-Xaa₂-G-E-Xaa₃)ₙ₂-Yaa₂-(G-Xaa₁-Xaa₂-G-E-Xaa₃)ₙ₃-Yaa₃-(G-Xaa₁-Xaa₂-G-E-Xaa₃)ₙ₃- Yaa₄-(G-Xaa₁-Xaa₂-G-E-Xaa₃)ₙ₄-............-Yaaₙ-(G-Xaa₁-Xaa₂-G-E-Xaa₃)ₙ-Yaaₙ₊₁-G-Xaa₁-Xaa₂-]_{N}

Wherein, the Xaa₁ is a non-polar hydrophobic amino acid; the Xaa₂ is one of serine (S), alanine (A), proline (P), and hydroxyproline (O); and the Xaa₃ is an alkaline amino acid;
Yaa₁, Yaa₂, Yaa₃, Yaa₄, ........., Yaaₙ, and Yaaₙ₊₁ at each occurrence are each independently selected from the group consisting of absence, one or more different or identical amino acids;
n2, n3, n4,..., and n is an integer greater than 0, and two of which are not 0 at the same time.

Preferably, the recombinant collagen sequence comprises no protein tag.

Preferably, the amino acid sequence of the recombinant collagen is SEQID NO.1.

Preferably, the anhydride is one of cyclic unsaturated anhydride or carboxyl-containing unsaturated anhydride or 4-pentenoic anhydride, crotonic anhydride and methacrylic anhydride; further, the anhydride is one of maleic anhydride, citraconic anhydride, cis-aconitic anhydride, 4-pentenoic anhydride, crotonic anhydride and methacrylic anhydride; and still further, the anhydride is methacrylic anhydride.

Preferably, the natural polysaccharide is selected from the group consisting of hyaluronic acid, carboxymethyl cellulose, methyl cellulose, hydroxyethyl cellulose, hydroxypropyl cellulose, alginic acid, glucan, agarose, heparin, chondroitin sulfate, ethylene glycol chitosan, propylene glycol chitosan, chitosan lactate, carboxymethyl chitosan or chitosan quaternary ammonium salt; preferably, this natural polysaccharide is carboxymethylcellulose.

Preferably, the activating agent is NHS and EDC; Further, the amino vicinal-diol is 3-amino-1,2-propanediol. By introducing a side chain group containing vicinal-diol, aldehyde group can be generated via specific oxidation, effectively avoiding the destruction of the main chain structure of a natural polysaccharide.

The EDC in the present invention refers to 1-(3-dimethylaminopropyl)-3-ethylcarbodiimide hydrochloride, and NHS refers to N-hydroxysuccinimide.

Further, the oxidizing agent is selected from the group consisting of periodate and hypochlorite; preferably, the oxidizing agent is sodium periodate.

In a second aspect, the present invention provides a preparation method of a transdermal photocuring forming hydrogel with biological activity, and the hydrogel is obtained by a transdermal photocuring method, as defined in claims 10-14,

Further, the specific steps are as follows:
S1, An appropriate chemical modification is applied to a natural polysaccharide using an anhydride to obtain a natural polysaccharide derivative with photo-crosslinking properties;
S2, mixing the above natural polysaccharide derivative with photo-crosslinking properties and an activating agent for reaction, the pH value of the reaction solution is 4.65 to -5.2;
S3, adding amino vicinal-diol to the reaction mixture of S2 for side chain carboxyl activation reaction, purifying the resulting reaction mixture by dialysis, after which freeze drying the solution to obtain a natural polysaccharide derivative containing a vicinal-diol side chain;
S4, dissolving the above natural polysaccharide derivative containing a vicinal-diol side chain and then reacting with an oxidizing agent, purifying the resulting reaction mixture by dialysis, after which freeze drying the solution to obtain a natural polysaccharide derivative containing an aldehyde side chain; and
S5, dissolving the above natural polysaccharide derivative containing an aldehyde side chain and then mixing it with the aforementioned recombinant collagen and a photoinitiator to obtain a repair matrix precursor solution, subjecting the mixture to transdermal photocrosslinking to obtain the hydrogel.

The present invention regulates the composition and structure of the natural polysaccharide through appropriate chemical modification and reasonable formula. Upon chemical modification, a photoinitiating group is introduced into the natural polysaccharide, so that it can be subcutaneously injected to achieve the transdermal photocuring forming effect, and its mechanical and degradation properties are also significantly improved; such special structure can also support the survival and proliferation of cells, making the finally obtained tissue filling agent (hydrogel) with good thermal stability, mechanical strength, anti-enzymolysis properties and longer maintenance time in vivo.

In the field of soft tissue filling, carboxymethyl cellulose, like other polysaccharide macromolecule compounds, lacks cell adhesion sites, which makes it difficult for cells to adhere and spread in the hydrogel, leading to the difficulty for cells to migrate and grow in the hydrogel, and thus they can only proliferate and aggregate in situ. The present invention makes full use of the biological safety, high adhesive activity and biological effect of the recombinant collagen, which is conducive to the adhesion and growth of cells on the compound hydrogel. With the degradation of carboxymethyl cellulose, the recombinant collagen is continuously released, which regulates the biological behavior of cells and induces the expression of extracellular matrix, so as to achieve the repair effect of morphological maintenance in early stage and induction of soft tissue matrix regeneration in later stage.

The hydrogel of the present invention can be used to improve the contour defects of soft tissue, and is suitable for the filling of the dermis layer (such as the middle and deep layers) and the subcutaneous superficial to deep layers, for example restoration of skin subsidence areas such as wrinkles, scars, depressed defects or injuries, etc.

Preferably, the photo-crosslinking modification degree of the natural polysaccharide derivative in the step S1 is 30% to 60%; and the molar ratio is 1: (1-3): (2-5) (the natural polysaccharide derivative with photo-crosslinking properties: NHS: EDC);
Further, in the step S2, the molar ratio is 1: 2: 3 (the natural polysaccharide derivative with photo-crosslinking properties: NHS: EDC);
Preferably, in the step S3, the pH value of the solution for the activation reaction to which an amino vicinal-diol is added is 7 to 7.7, and the molar ratio is (1-10): 1 (amino vicinal-diol: the natural polysaccharide derivative with photo-crosslinking properties); the dialysis time in the S3 and S4 is 20 hours to 72 hours, and the molecular weight retained by dialysis is 8000 kD to 14000 kDa.

The natural polysaccharide derivative of the present invention contains an aldehyde group, which can be combined with the recombinant collagen through the Schiff's reaction and gradually released in the hydrogel. Preferably, in the step S5, the mass-to-volume ratio of the natural polysaccharide derivative containing an aldehyde side chain is 1% to 5%; the mass-to-volume ratio of the recombinant collagen is 0.5% to 2%; and the mass-to-volume ratio of the photoinitiator is 0.01% to 0.05%.

In a third aspect, the present invention provides a non therapeutic application of the transdermal photocuring forming hydrogel with biological activity in soft tissue filling and repair.

The hydrogel of the present disclosure can also be used together with other functional materials as required, such as ingredients with antibacterial and bacteriostatic effects, anti-aging ingredients, anticoagulant ingredients, antioxidant ingredients, and growth factors, etc.

All features disclosed in this specification, or all methods or steps in the process disclosed herein can be combined in any way, except for mutually exclusive features and/or steps.

When the mass-to-volume ratio or other values or parameters are represented by a range, a preferred range, or a range limited by a series of upper-and lower-limit preferred values, it should be understood as specifically disclosing all ranges formed by any combination of any upper limits or preferred values of the range with any lower limits or preferred values of the range, regardless of whether the range is separately disclosed. For example, when the scope "1 to 5" is disclosed, the described scope should be interpreted as including ranges "1 to 4", "1 to 3", "1 to 2", "1 to 2 and 4 to 5", and "1 to 3 and 5", etc. When a numerical range is described herein, it is intended to include its end value and all integers and fractions within that range, unless otherwise specified.

The present invention has the following beneficial effects:
(1) In the present invention, the natural polysaccharide is firstly chemically modified with an unsaturated anhydride to introduce unsaturated carbon-carbon double bonds, and then the carboxyl group in the modified natural polysaccharide is activated through EDC/NHS, subsequently an amino vicinal-diol is added to introduce an adjacent hydroxyl group, which is oxidized by an oxidizing agent to obtain an aldehyde group, and finally the aldehyde group undergoes a Schiff's reaction with the amino group in the recombinant collagen, and the soft tissue filling hydrogel activated by the recombinant collagen is obtained by transdermal photo-crosslinking. The hydrogel obtained in the present invention has good biocompatibility, is easy to operate, and has the characteristics of injectability and in-situ curing and shaping, which also meet the clinical filling requirements on irregular defective tissues.
(2) The present invention is based on natural polysaccharide materials such as carboxymethyl cellulose, etc. It only requires three steps: material pre-mixing, local-injection, and photo-curing to construct a novel compound tissue filling agent. The preparation method is simple to operate, the degree of modification reaction is controllable, and the defect of lacking cell adhesion sites in natural polysaccharide materials such as carboxymethyl cellulose, etc. is solved.
(2) The photo-initiation method of the present invention is simple to operate with a mild reaction condition. The method applied is transdermal photo-crosslinking, which requires no destructive operation. The surgery has the advantages like shaping during the operation and forming immediately after that.

### DESCRIPTION OF DRAWINGS

The accompanying drawings illustrated here are used to provide a further understanding of the present invention, which form a part of the present invention. The illustrative embodiment and their descriptions of the present invention are used to explain the present invention, and do not constitute an improper limitation thereto. In the accompanying drawings:
Figure 1 shows the cytotoxicity assay results of natural polysaccharide derivatives (CMC-MA) with photo-crosslinking properties;
Figure 2 shows the morphology and mechanical strength of CMC-MA hydrogel; A: the gelation morphology of 2% CMC-MA material under different in vitro transdermal light illumination time points, B: the storage modulus, loss modulus, and Tan Delta of the gelation of 2% CMC-MA material under different in vitro transdermal light illumination time points;
Figure 3 shows the gelation conditions of the recombinant collagen activated transdermal photocuring forming soft tissue filling agent (CMC-MA-AP-CHO-rhCol III) in SD rats after subcutaneous injection and before & after transdermal light illumination;
Figure 4 shows the release curve of recombinant collagen, A: rhCol III release curve of CMC-MA/rhCol III hydrogel at different time points, B: rhCol III release curve of CMC-MA-AP-CHO-rhCol III hydrogel at different time points; and
Figure 5 shows the CMC-MA-AP-CHO-rhCol III loading force test, with Sample 1, Sample 2, and Sample 3 representing samples on which the test is repeated three times

### Detailed Description of Embodiments

In order to make the object, technical solution, and advantages of the present invention clearer and more obvious, the present invention will be further explained below in detail in conjunction with embodiment. It should be understood that the specific embodiment described here are only intended to explain the present invention and are defined by the scope of the claims.

Unless otherwise defined, all technical and scientific terms used herein have the same meaning as those commonly understood by those skilled in the technical field of the present invention. The terms used in the specification of the present invention are only for the purpose of describing specific embodiment.

It should be clarified that the experimental methods used in the following embodiment are all conventional methods, unless otherwise specified. The materials and reagents, etc. used in the following embodiment are commercially available, unless otherwise specified.

The terms "comprising", "including", "having", "containing" or any other variation thereof used herein are intended to cover non-exclusive inclusion. For example, compositions, steps, methods, or articles containing the listed elements need not be limited to those elements, but can include other elements that are not explicitly listed or inherent elements in such compositions, steps, methods, or articles.

### Embodiment 1

### (1) Preparation of transdermal photocuring forming soft tissue filling matrix

Modification of carboxymethyl cellulose (CMC, 250KD, DS=0.7) using methacrylic anhydride (MA): Weight 1 g of raw material CMC and dissolve in 100 mL of deionized water to a concentration of 1% (wt), the mixture is stirred for 6 hours with a mechanical stirrer and then placed in a refrigerator at 4°C for 24 hours, then stir at room temperature for 2 hours to have well dissolved solution and the molecular chain is fully extended, which is conducive to the modification reaction. The dissolved 1% CMC solution is placed in a low-temperature water bath controlled by a circulating pump, and the reaction temperature is kept for 2 hours. At the beginning of the reaction, 2 mL of methacrylic anhydride is added dropwise to the CMC solution. During the reaction, the pH is adjusted with 5M NaOH to maintain the value between 8.0 and 8.5, after the pH of the reaction solution is stabilized, the solution is reacted overnight for 24 hours. Pour the reaction solution into 500 mL of ethanol to obtain the precipitate product, filter and remove some unreacted methacrylic anhydride and byproducts at the same time. Wash the product with ethanol for three times, then transfer into a dialysis bag of 8000 kDa to 14000 kDa and dialyzed with deionized water for 4 days, with the water changed three times a day. After the dialysis, proceed with freeze-dried to obtain the natural polysaccharide derivative CMC-MA with photo-crosslinking properties, store the product at 4°C.

### (2) Preparation of recombinant collagen

### 1. Preparation of recombinant collagen by Escherichia coli. Fermentation

Gene recombination and transcription: Collagen DNA fragments are subjected to codon optimization and splice recombination using a PCR method. The expression vector is constructed using pET-32a and then transferred to the *Escherichia coli.* expression strain BL21. After cultivation and screening, *Escherichia coli.* genetic engineering strains with high protein expression are obtained.

Fermentation culture: the optimized single colony of *Escherichia coli.* genetic engineering strains is selected from LB plate and placed in a 100 mL Erlenmeyer flask containing 10 mL of LB medium, cultured at 37°C and 220 rpm for 12 hours to 16 hours, the bacterial solution is inoculated into a fermentation tank containing LB medium at a volume ratio of 1:100 for scale-up cultivation, and cultured at 37°C and 220 rpm for 3 hours until the OD600 is about 0.6, then add 0.5 mM of IPTG, and culture at 16°C for 20 hours, centrifuge to collect the thalli.

Protein separation and purification: suspend the thalli with tris buffer, stir at high speed to completely dissolve the thalli, then centrifuge, the supernatant is collected and cooled to 4°C and then filtered with 1 µm, 0.45 µm and 0.22 µm of membrane sequentially, and further purified by affinity chromatography to obtain the recombinant collagen.

The characteristic of the amino acid sequence of recombinant collagen: a repetitive unit with a combination of multiple characteristic amino acid sequences, i.e. GER GAP GFR GPA GPN GIP GEK GPA GER GAP, which is connected 16 times to obtain the recombinant collagen of the present invention, and the amino acid sequence is shown in SEQID NO.1:

GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP
GERGAPGFRG PAGPNGIPGE KGPAGERGAP.

### 2. Cell culture method

1) Preparation of specimen:
   Prepare recombinant collagen solution having a certain concentration (such as 0.5 mg/mL) using PBS, and animal-derived collagen solution is to the same concentration (such as 0.5 mg/mL) using PBS, while PBS is used as the blank;
2) Plating: add the experimental solution to the 96-well plate, with 100 µL each well, each group consists 5 wells and stand overnight at 4°C;
3) Blocking: After the plating is completed, remove the liquid from the well and wash the well twice with 200 µL of PBS solution, and then add 100 µL of heat-inactivated 1% BSA-PBS solution and incubate at 37°C and 5% CO₂ for 1 hour;
4) Cell inoculation: After the incubation is completed, remove the liquid from the well and wash the well twice with 200 µL of PBS solution, add 100 µL of cell suspension with a concentration of 5×10⁴ to 1×10⁶ cells/mL to each well and incubate at 37°C and 5% CO₂ for 1 hour;
5) Testing: After the incubation is completed, remove the liquid from the well and wash the well twice with 200 µL of PBS solution, add 150 µL of CCK-8 and incubate at 37°C and 5% CO₂ for 1 hour, take 100 µL of solution and add to a new 96-well plate, then the absorbance value (OD) is measured at 450 nm.

### (3) Preparation of recombinant collagen activated transdermal photocuring forming soft tissue filling agent

(3.1) The methacrylated carboxymethyl cellulose CMC-MA, the natural polysaccharide derivative with photo-crosslinking properties, with a modification degree of 30% to 40% is dissolved in deionized water to obtain a 0.5% methacrylated carboxymethyl cellulose solution;
(3.2) Add a solid form of NHS and a solid form of EDC to the solution in sequence (with a molar ratio of CMC-MA:EDC:NHS at 1:2:3), and the pH of the mixed solution is maintained at 4.75 to 5 with 5.0 M of NaOH solution or 5.0M of HCl solution;
(3.3) Stir at room temperature and react for 2 hours;
(3.4) the pH of the reaction solution is adjusted to 7.4 with 1.0M of NaOH solution;
(3.5) Add 3-amino-1,2-propanediol (AP) to the reaction solution with an EDC concentration of 5 mM, so that the molar ratio is 5:1 (3-amino-1,2-propanediol: methacrylated carboxymethyl cellulose) in the mixed solution, and stir for 24 hours;
(3.6) The reaction solution is transferred into a dialysis bag with a molecular weight retain of 8000 to 14000. After three days of dialysis with deionized water, proceed with freeze-drying to obtain a natural polysaccharide derivative CMC-MA-AP containing a vicinal-diol chain;
(3.7) Dissolve the freeze-dried sponge-like product from step (3.6) in deionized water, drop 0.5M sodium periodate solution to the solution, with a molar ratio of 0.2:1 (0.5M sodium periodate: CMC-MA-AP), stir for 24 hours;
(3.8) Transfer the reaction solution into a dialysis bag with a molecular weight retain of 8000 to 14000. After three days of dialysis with deionized water, proceed with freeze-drying to obtain a natural polysaccharide derivative CMC-MA-AP-CHO containing an aldehyde chain;
(3.9) Dissolve the CMC-MA-AP-CHO obtained by freeze-drying in step (3.8) in deionized water to obtain a concentration of 2%, add recombinant humanized type III collagen (rhCol III) with a final concentration of 1%, and LAP photoinitiator solution (with a final concentration of 0.02% by mass-to-volume ratio).
(3.10) Transfer the precursor solution from step (3.9) in a silicone mold with a specification of Φ6 mm×2.5 mm, which is exposed for 1 minute under ultraviolet light, to obtain the recombinant collagen activated transdermal photocuring forming soft tissue filling agent CMC-MA-AP-CHO-rhCol III hydrogel.

### Test example 1: Cytotoxicity assay of CMC-MA and CMC-MA-AP-CHO-rhColIII hydrogel

### Cytotoxicity assay of CMC-MA

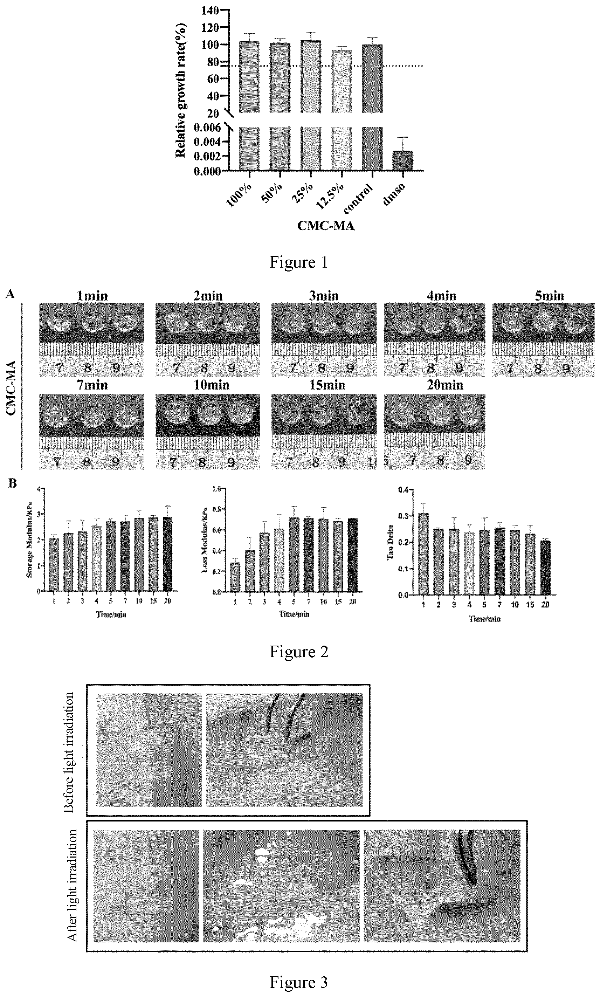

1. Preparation of material extraction solution: After sterilization of CMC-MA, prepare a solution at concentration of 2%. Then, fill the solution in a syringe under sterile conditions, and the solidified material is prepared under sterile conditions, with a dimensions Φ=8±0.1mm and h=2.0 ± 0.2mm. Put the material d in a 48-well plate and α-MEM culture medium is added according to the recommended ratio of 0.2 g/mL in GB/T 16886.12-2017, *Biological Evaluation of Medical Devices - Part 12, Sample Preparation and Reference Materials,* to completely immerse the compound material and then stand in an incubator at 37°C and 5% CO₂ for 24 hours, then take the supernatant, add an appropriate volume of to 10% newborn fetal bovine serum (FBS) to obtain a series of solutions with concentrations of 100%, 50%, 25%, and 12.5%, respectively.
2. L929 cells are thawed for culture and passage to P3 generation, the cells are digested, and adjusted to a concentration of 5×10⁴ cells/mL, the cell suspension is pipetted and added to a 96-well plate, 100 µL per well, then cultured for 24 hours using 10% newborn fetal bovine serum in α-MEM as the culture medium. Then discard culture medium, add 150 µL of sample extraction solutions with concentrations of 100%, 50%, 25%, and 12.5%, 20% DMSO (positive control), and culture medium containing 10% FBS (negative control), respectively. At the same time, add a culture medium containing 10% FBS to the peripheral wells as a blank control. 5 parallel samples are used for each group of specimens and incubate for 24 hours.
3. After incubation for 24 hours, discard the supernatant and add 150 µL of CCK-8 solution to each well, incubate in darkness for 2 hours. Pipette 100 µL of supernatant per well and add to a new 96-well plate, measure the optical density value (OD value) at 450 nm using a microplate reader. Calculate cell survival rate and according to the data analysis method in ISO 10993-5:2017, Appendix C. The results are shown in **Figure 1****.**

According to the provisions on cytotoxicity in GB/T 16886.5-2017, *Biological Evaluation of Medical Devices - Part 5: in vitro Cytotoxicity Assay,* the safety of CMC-MA compound materials is acceptable.

Cytotoxicity assay of CMC-MA-AP-CHO-rhColIII hydrogel CMC-MA-AP-CHO-rhCol III hydrogel is tested for toxicity according to the method in *CMC-MA cytotoxicity Assay.* According to the provisions on cytotoxicity in GB/T 16886.5-2017, *Biological Evaluation of Medical Devices - Part 5: in vitro Cytotoxicity Assay,* the safety of hydrogel compound materials is acceptable .

### Test Example 2: In vitro subcutaneous transdermal photogelation of CMC-MA in SD rats

1. Fill 2% of CMC-MA solution into a 1 mL syringe and the compound material is injected into the mold (d: 8 mm, h: 2 mm). Using 12-week old rats, take the back skin and remove the hair, put the materials in the mold under the skin in vitro, and illuminate with a 5W blue-light flashlight (wavelength 405 nm) for different period of time (1, 2, 3, 4, 5, 7, 10, 15, and 20 minutes).
2. Remove the mold, photograph immediately and measure the mechanical strength (storage modulus, and loss modulus) of the hydrogel through Dynamic mechanical analysis (DMA). As shown in **Figure 2****,** after 1 minute of transdermal illumination, the gelatin is formed. The mechanical strength of hydrogel gradually increased with the extension of transdermal illumination time. When the illumination time is more than 5 minutes, the strength of hydrogel shows no significant statistical difference.

### Test Example 3: In vivo subcutaneous photogelation of CMC-MA-AP-CHO-rhCol III in SD rats

1. Fill 2% of CMC-MA-AP-CHO-rhCol III solution into a 1 mL syringe, take the back skin of rats and remove the hair. Then 0.2 mL of CMC-MA-AP-CHO-rhCol III solution iss subcutaneously injected and shaped appropriately, the change in the back skin state before light illumination and the state of material under skin before illumination are recorded **(****Figure 3****).**
2. A 5W blue-light flashlight (wavelength 405nm) is used to illuminate the skin for a period of time (1 minute), and the state of the skin and the gelation of the material under skin after illumination are recorded **(****Figure 3****).**

It can be seen from **Figure 3** that after transdermal injection, the material does not form gelatin and appears in a sticky state without light illumination. After 1 minute of transdermal illumination, the material change to gelatin obviously and appears an obvious fixed form.

**Test Example 4: CMC-MA-AP-CHO-rhCol III hydrogel protein release test**
1. Fill 2% of CMC-MA-AP-CHO-rhCol III (rhCol III with a final concentration of 10 mg/mL and CMC-MA/rhCol III (rhCol III with a final concentration of 10 mg/mL solutions into 1 mL syringes respectively, and the compound materials are injected into the mold (d: 8 mm, h: 2 mm). A 5W blue-light flashlight (wavelength 405nm) is used to illuminate for 1 minute. The mass of CMC-MA-AP-CHO-rhCol III and CMC-MA/rhCol III hydrogels is accurately weighed, and the volume of extraction solution is determined according to the ratio of 0.2 g/mL (refer to GB/T 16886.12). The hydrogel is immersed into the extraction solution and placed in a constant temperature shaker (37°C, 70 rpm/min), make three parallel samples for each group, 100 µl of extraction solution is collected from each parallel sample at the predetermined time point and 100 µL of fresh extraction solution is added each time.
2. Test concentration of rhCol III in the extraction solution(at 1, 2, 4, 6, 8, 12, 24, 36, 48 and 72 hours) using Bradford method . The absorbance value (Abs) is detected at a wavelength of 595nm by a UV-Vis spectrophotometer. Based on the concentration of standard protein solution and the corresponding absorbance value, a standard curve is drawn, the protein content in the test solution is then calculated. Please refer to the instruction manual (Solarbio, PC0015) for specific operations.

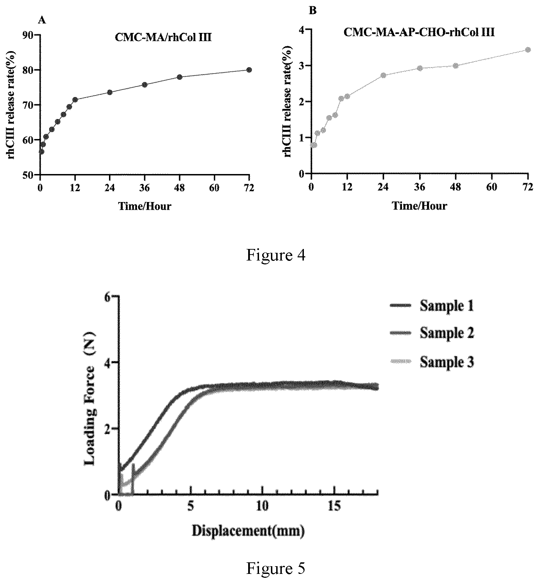

The results are shown in **Figure 4****.**As can be seen from **Figure 4A****,** the release rate of rhCol III from CMC-MA/rhCol III hydrogel gradually increases with time, at 24 h, the release rate of rhCol III reaches a maximum of 72.35%, and shows a continuously increasing trend, reaching 80% in 72 hours. As can be seen from **Figure 4B****,** the release rate of CMC-MA-AP-CHO-rhCol III hydrogel is only 2.73% at 24 h, and the release rate of rhCol III slowly increases with time. It can be seen that the hydrogel prepared by the method of the present invention can achieve the effect of sustained release of recombinant collagen, effectively solving the problem of burst release of recombinant collagen when it is used alone, shortening the treatment time, and having a long-lasting effect.

### Test Example 5: CMC-MA-AP-CHO-rhCol III loading force test

The injectability of injectable materials is related to the convenience for surgeon's use. And the presence of obstacles such as blockages and discontinuities, etc. in the extrusion process or not, is an important physicochemical indicator. To evaluate the injectable performance, the loading force of compound materials in the syringe is measured with a universal material testing machine (AGS-X, SHIMADZU, Japan), and the specific method is as follows:
1. Sample preparation: According to the expected technical requirements for injectable compound materials, fill 2% of CMC-MA-AP-CHO-rhCol III precursor solution into a 1 mL syringe equipped with a 30-gauge needle, and preserve it at 4°C;
2. Pre-preparation: Before the test, remove the air in the front end of the syringe needle, then push the piston until the material is squeezed out.
3. Testing: the syringe filled with materials is fixed in a special apparatus, and a universal material testing machine is used to vertically compress the piston and push, with a constant speed of 30 mm/min, the loading force versus movement distance curve is recorded, and the test is repeated three times for each sample.

As shown in **Figure 5****,** the driving force of the 2% of CMC-MA-AP-CHO-rhCol III precursor solution reaches a relatively stable level after a rapid linear increase until the material is completely pushed out. In the constant pushing stage, the driving force is maintained at 2.76 ± 0.96 N, and the curve shows horizontal with little changes, indicating that the material formula has uniform composition without obvious bubbles. At the same time, the material needs low loading force, having good injectability, and is easy to use for surgeons.

In summary, the natural polysaccharide derivatives prepared in the present invention exhibit significant gelation in vitro and in vivo upon transdermal injection and light illumination for 1 minute, having quick gelation time and no damage to skin tissues. The natural polysaccharide derivative with photo-crosslinking properties CMC-MA reacts with 3-amino-1,2-propanediol and is oxidized to obtain a natural polysaccharide derivative CMC-MA-AP-CHO, which reduces the damage to the main chain of the sugar ring during the oxidation process, and can reduce the decline of mechanical strength to a certain extent. At the same time, the test results also show that the hydrogel of such modified natural polysaccharide derivative shows good safety and also has good injectability and operability. With the degradation of carboxymethyl cellulose, the recombinant collagen activated CMC-MA-AP-CHO-rhCol III hydrogel can continuously release rhCol III, and induce the biological behavior of extracellular matrix, thus achieve the repair effect of morphological maintenance in early stage and induction of soft tissue matrix regeneration in later stage, which has a more long-lasting action time

The aforementioned examples are only illustrative and are used to explain some features of the method described in the present invention.

## Claims

1. A transdermal photocuring forming hydrogel with biological activity, is **characterized in that**, the raw materials of which comprise a recombinant collagen, an anhydride, a natural polysaccharide, an activating agent, an amino vicinal-diol and an oxidizing agent;
The amino acid sequence of the recombinant collagen comprises N basic repetitive units, and each basic repetitive unit contains n1 of the following characteristic amino acid sequences: "G-Xaa₁-Xaa₂-G-E-Xaa₃"; the 3' end and the 5' end of the basic repetitive unit are connected to form the above characteristic amino acid sequence;
Wherein, the Xaa1 is a non-polar hydrophobic amino acid; the Xaa2 is one of serine (S), alanine (A), proline (P), and hydroxyproline (O); and the Xaa3 is an alkaline amino acid; N is an integer greater than 4; and n1 is an integer greater than 3.

2. The hydrogel according to claim 1, is **characterized in that**, N is an integer ranging from 4 to 300.

3. The hydrogel according to claim 1, is **characterized in that**, N is an integer ranging from 4 to 200.

4. The hydrogel according to claim 1, is **characterized in that**, the characteristic amino acid sequences are arranged in the basic repetitive unit continuously or at intervals.

5. The hydrogel according to claim 1, is **characterized in that**, the amino acid sequence of the recombinant collagen presents the following characteristics:
[-G-E-Xaa₃-Yaa₁-(G-Xaa₁-Xaa₂-G-E-Xaa₃)ₙ₂-Yaa₂-(G-Xaa₁-Xaa₂-G-E-Xaa₃)ₙ₃-Yaa₃-(G-Xaa₁-Xaa₂-G-E-Xaa₃)ₙ₃- Yaa₄-(G-Xaa₁-Xaa₂-G-E-Xaa₃)ₙ₄-............-Yaaₙ-(G-Xaa₁-Xaa₂-G-E-Xaa₃)ₙ-Yaaₙ₊₁-G-Xaa₁-Xaa₂-]_{N}
Wherein,
Yaa₁, Yaa₂, Yaa₃, Yaa₄, ........., Yaaₙ, and Yaaₙ₊₁ at each occurrence are independently selected from the group consisting of absence, one or more different or identical amino acids;
n2, n3, n4, ..., and n is an integer greater than 0, and two of which are not 0 at the same time.

6. The hydrogel according to claim 1, **characterized in that**, the recombinant collagen sequence comprises no protein tag.

7. The hydrogel according to claim 1, is **characterized in that**, the amino acid sequence of the recombinant collagen is SEQID NO.1.

8. The hydrogel according to claim 1, is **characterized in that**, the anhydride is one of cyclic unsaturated anhydride or carboxyl-containing unsaturated anhydride or 4-pentenoic anhydride, crotonic anhydride and methacrylic anhydride.

9. The hydrogel according to claim 1, is **characterized in that**, the anhydride is one of maleic anhydride, citraconic anhydride, cis-aconitic anhydride, 4-pentenoic anhydride, crotonic anhydride and methacrylic anhydride;
the natural polysaccharide is selected from hyaluronic acid, carboxymethyl cellulose, methyl cellulose, hydroxyethyl cellulose, hydroxypropyl cellulose, alginic acid, glucan, agarose, heparin, chondroitin sulfate, ethylene glycol chitosan, propylene glycol chitosan, chitosan lactate, carboxymethyl chitosan or chitosan quaternary ammonium salt;
the activating agent is NHS and EDC; the amino vicinal-diol is 3-amino-1,2-propanediol; and the oxidizing agent is selected from periodate and hypocholoride; Further,
the oxidizing agent is sodium periodate.

10. A preparation method of the transdermal photocuring forming hydrogel in claim 1, is **characterized in that**, the hydrogel is obtained by a transdermal photocuring method, and the specific steps are as follows:
S1, an appropriate chemical modification is applied to a natural polysaccharide using an anhydride to obtain a natural polysaccharide derivative with photo-crosslinking properties;
S2, mixing the above natural polysaccharide derivative with photo-crosslinking properties and an activating agent for reaction, the pH value of the reaction solution is 4.65 to 5.2;
S3, adding amino vicinal-diol to the reaction system of S2 for side chain carboxyl activation reaction, purifying the resulting reaction mixture by dialysis, after which freeze drying the resulting solution to obtain a natural polysaccharide derivative containing a vicinal-diol side chain;
S4, dissolving the above natural polysaccharide derivative containing a vicinal-diol side chain and then reacting with an oxidizing agent, purifying the resulting reaction mixture by dialysis, after which freeze drying the solution to obtain a natural polysaccharide derivative containing an aldehyde side chain; and
S5, dissolving the above natural polysaccharide derivative containing an aldehyde side chain and then mixing it with the recombinant collagen and a photoinitiator to obtain a repair matrix precursor solution, subjecting the mixture to transdermal photocrosslinking to obtain the hydrogel.

11. The preparation method according to claim 10, is **characterized in that**, the modification degree of the natural polysaccharide derivative with photo-crosslinking properties in the step S1 is 30% to 60%; with a molar ratio of 1: (1-3): (2-5) (the natural polysaccharide derivative with photo-crosslinking properties: NHS: EDC).

12. The preparation method according to claim 10, is **characterized in that**, in the step S2, the molar ratio is 1: 2: 3 (the natural polysaccharide derivative with photo-crosslinking properties: NHS: EDC).

13. The preparation method according to claim 10, is **characterized in that**, in the step S3, the pH value of the solution for the activation reaction to which an amino vicinal-diol is added is 7 to 7.7, and the molar ratio is (1-10): 1 (amino vicinal-diol : the natural polysaccharide derivative with photo-crosslinking properties); the dialysis time in the S3 and S4 is 20 hours to 72 hours, and the molecular weight retained by dialysis is 8000 kD to 14000 kDa.

14. The preparation method according to claim 10, is **characterized in that**, in the step S5, the mass-to-volume ratio of the natural polysaccharide derivative containing an aldehyde side chain is 1% to 5%; the mass-to-volume ratio of the recombinant collagen is 0.5% to 2%; and the final concentration mass-to-volume ratio of the photoinitiator is 0.01% to 0.05%.

15. A non-therapeutic application of the transdermal photocuring forming hydrogel with biological activity of claim 1 in soft tissue filling and repair.

## Patentansprüche

1. Durch transdermale Photohärtung gebildetes Hydrogel mit biologischer Aktivität, **dadurch gekennzeichnet, dass** dessen Ausgangsstoffe ein rekombinantes Kollagen, ein Anhydrid, ein natürliches Polysaccharid, ein Aktivierungsmittel, ein vicinales Aminodiol und ein Oxidationsmittel umfassen; wobei die Aminosäuresequenz des rekombinanten Kollagens N grundlegende repetitive Einheiten umfasst und jede grundlegende repetitive Einheit n1 der folgenden charakteristischen Aminosäuresequenzen enthält: "G-Xaa₁-Xaa₂-G-E-Xaa₃"; wobei das 3'-Ende und das 5'-Ende der grundlegenden repetitiven Einheit verbunden sind, um die obige charakteristische Aminosäuresequenz zu bilden; wobei Xaa₁ eine unpolare hydrophobe Aminosäure ist; Xaa₂ eines aus Serin (S), Alanin (A), Prolin (P) und Hydroxyprolin (O) ist; und Xaa₃ eine basische Aminosäure ist; N eine ganze Zahl größer als 4 ist; und n1 eine ganze Zahl größer als 3 ist.

2. Hydrogel nach Anspruch 1, **dadurch gekennzeichnet, dass** N eine ganze Zahl im Bereich von 4 bis 300 ist.

3. Hydrogel nach Anspruch 1, **dadurch gekennzeichnet, dass** N eine ganze Zahl im Bereich von 4 bis 200 ist.

4. Hydrogel nach Anspruch 1, **dadurch gekennzeichnet, dass** die charakteristischen Aminosäuresequenzen in der grundlegenden repetitiven Einheit kontinuierlich oder in Intervallen angeordnet sind.

5. Hydrogel nach Anspruch 1, **dadurch gekennzeichnet, dass** die Aminosäuresequenz des rekombinanten Kollagens die folgenden Eigenschaften aufweist: [-G-E-Xaa₃-Yaa₁-(G-Xaa₁-Xaa₂-G-E-Xaa₃)ₙ₂-Yaa₂-(G-Xaa₁-Xaa₂-G-E-Xaa₃)ₙ₃-Yaa₃-(G-Xaa₁-Xaa₂-G-E-Xaa₃)ₙ₃-Yaa₄-(G-Xaa₁-Xaa₂-G-E-Xaa₃)ₙ₄-.........-Yaaₙ-(G-Xaa₁-Xaa₂-G-E-Xaa₃)ₙ-Yaaₙ₊₁-G-Xaa₁-Xaa₂-]N wobei: Yaa₁, Yaa₂, Yaa₃, Yaa₄, ........, Yaaₙ und Yaaₙ₊₁ bei jedem Auftreten jeweils unabhängig ausgewählt sind aus der Gruppe bestehend aus Abwesenheit, einer oder mehreren unterschiedlichen oder identischen Aminosäuren; n2, n3, n4, ..., und n ganze Zahlen größer als 0 sind, und zwei davon nicht gleichzeitig 0 sind.

6. Hydrogel nach Anspruch 1, **dadurch gekennzeichnet, dass** die Sequenz des rekombinanten Kollagens kein Protein-Tag umfasst.

7. Hydrogel nach Anspruch 1, **dadurch gekennzeichnet, dass** die Aminosäuresequenz des rekombinanten Kollagens SEQ ID NO: 1 ist.

8. Hydrogel nach Anspruch 1, **dadurch gekennzeichnet, dass** das Anhydrid eines aus zyklischem ungesättigtem Anhydrid oder carboxylhaltigem ungesättigtem Anhydrid oder 4-Pentensäureanhydrid, Crotonsäureanhydrid und Methacrylsäureanhydrid ist.

9. Hydrogel nach Anspruch 1, **dadurch gekennzeichnet, dass**: das Anhydrid eines aus Maleinsäureanhydrid, Citraconsäureanhydrid, cis-Aconitsäureanhydrid, 4-Pentensäureanhydrid, Crotonsäureanhydrid und Methacrylsäureanhydrid ist; das natürliche Polysaccharid ausgewählt ist aus Hyaluronsäure, Carboxymethylcellulose, Methylcellulose, Hydroxyethylcellulose, Hydroxypropylcellulose, Alginsäure, Glucan, Agarose, Heparin, Chondroitinsulfat, Ethylenglykol-Chitosan, Propylenglykol-Chitosan, Chitosanlactat, Carboxymethyl-Chitosan oder quaternärem Ammoniumsalz von Chitosan; das Aktivierungsmittel NHS und EDC ist; das vicinale Aminodiol 3-Amino-1,2-propandiol ist; und das Oxidationsmittel ausgewählt ist aus Periodat und Hypochlorit; ferner das Oxidationsmittel Natriumperiodat ist.

10. Herungsverfahren für das durch transdermale Photohärtung gebildete Hydrogel nach Anspruch 1, **dadurch gekennzeichnet, dass** das Hydrogel durch ein transdermales Photohärtungsverfahren erhalten wird und die spezifischen Schritte wie folgt sind:
S1, eine geeignete chemische Modifikation wird an einem natürlichen Polysaccharid unter Verwendung eines Anhydrids durchgeführt, um ein Derivat eines natürlichen Polysaccharids mit photovernetzenden Eigenschaften zu erhalten;
S2, Mischen des obigen Derivats eines natürlichen Polysaccharids mit photovernetzenden Eigenschaften und eines Aktivierungsmittels zur Reaktion, wobei der pH-Wert der Reaktionslösung 4,65 bis 5,2 beträgt;
S3, Zugeben von vicinalem Aminodiol zu dem Reaktionssystem von S2 für eine Seitenketten-Carboxyl-Aktivierungsreaktion, Aufreinigen des resultierenden Reaktionsgemischs durch Dialyse, woraufhin die resultierende Lösung gefriergetrocknet wird, um ein Derivat eines natürlichen Polysaccharids zu erhalten, das eine vicinale Diol-Seitenkette enthält;
S4, Lösen des obigen Derivats eines natürlichen Polysaccharids, das eine vicinale Diol-Seitenkette enthält, und anschließendes Reagieren mit einem Oxidationsmittel, Aufreinigen des resultierenden Reaktionsgemischs durch Dialyse, woraufhin die Lösung gefriergetrocknet wird, um ein Derivat eines natürlichen Polysaccharids zu erhalten, das eine Aldehyd-Seitenkette enthält; und
S5, Lösen des obigen Derivats eines natürlichen Polysaccharids, das eine Aldehyd-Seitenkette enthält, und anschließendes Mischen desselben mit dem rekombinanten Kollagen und einem Photoinitiator, um eine Reparaturmatrix-Vorläuferlösung zu erhalten, Unterziehen der Mischung einer transdermalen Photovernetzung, um das Hydrogel zu erhalten.

11. Herstellungsverfahren nach Anspruch 10, **dadurch gekennzeichnet, dass** der Modifikationsgrad des Derivats eines natürlichen Polysaccharids mit photovernetzenden Eigenschaften im Schritt S1 30 % bis 60 % beträgt; mit einem Molverhältnis von 1 : (1-3) : (2-5) (Derivat eines natürlichen Polysaccharids mit photovernetzenden Eigenschaften : NHS : EDC).

12. Herstellungsverfahren nach Anspruch 10, **dadurch gekennzeichnet, dass** im Schritt S2 das Molverhältnis 1 : 2 : 3 beträgt (Derivat eines natürlichen Polysaccharids mit photovernetzenden Eigenschaften : NHS : EDC).

13. Herstellungsverfahren nach Anspruch 10, **dadurch gekennzeichnet, dass** im Schritt S3 der pH-Wert der Lösung für die Aktivierungsreaktion, zu der ein vicinales Aminodiol zugegeben wird, 7 bis 7,7 beträgt und das Molverhältnis (1-10) : 1 beträgt (vicinales Aminodiol : Derivat eines natürlichen Polysaccharids mit photovernetzenden Eigenschaften); die Dialysezeit in S3 und S4 20 Stunden bis 72 Stunden beträgt und das durch Dialyse zurückgehaltene Molekulargewicht 8.000 kDa bis 14.000 kDa beträgt.

14. Herstellungsverfahren nach Anspruch 10, **dadurch gekennzeichnet, dass** im Schritt S5 das Masse-zu-Volumen-Verhältnis des Derivats eines natürlichen Polysaccharids, das eine Aldehyd-Seitenkette enthält, 1 % bis 5 % beträgt; das Masse-zu-Volumen-Verhältnis des rekombinanten Kollagens 0,5 % bis 2 % beträgt; und das Masse-zu-Volumen-Verhältnis der Endkonzentration des Photoinitiators 0,01 % bis 0,05 % beträgt.

15. Nicht-therapeutische Verwendung des durch transdermale Photohärtung gebildeten Hydrogels mit biologischer Aktivität nach Anspruch 1 bei der Weichgewebefüllung und - reparatur.

## Revendications

1. Hydrogel à formation par photodurcissement transdermique doté d'une activité biologique, **caractérisé en ce que** ses matières premières comprennent un collagène recombinant, un anhydride, un polysaccharide naturel, un agent d'activation, un diol vicinal aminé et un agent oxydant ; la séquence d'acides aminés du collagène recombinant comprend N motifs répétitifs de base, et chaque motif répétitif de base contient n1 des séquences d'acides aminés caractéristiques suivantes : « G-Xaa₁-Xaa₂-G-E-Xaa₃ » ; l'extrémité 3' et l'extrémité 5' du motif répétitif de base sont reliées pour former la séquence d'acides aminés caractéristique ci-dessus ; dans lequel Xaa₁ est un acide aminé hydrophobe non polaire ; Xaa₂ est l'un parmi la sérine (S), l'alanine (A), la proline (P) et l'hydroxyproline (O) ; et Xaa₃ est un acide aminé basique ; N est un nombre entier supérieur à 4 ; et n1 est un nombre entier supérieur à 3.

2. Hydrogel selon la revendication 1, **caractérisé en ce que** N est un nombre entier allant de 4 à 300.

3. Hydrogel selon la revendication 1, **caractérisé en ce que** N est un nombre entier allant de 4 à 200.

4. Hydrogel selon la revendication 1, **caractérisé en ce que** les séquences d'acides aminés caractéristiques sont disposées dans le motif répétitif de base de manière continue ou par intervalles.

5. Hydrogel selon la revendication 1, **caractérisé en ce que** la séquence d'acides aminés du collagène recombinant présente les caractéristiques suivantes : [-G-E-Xaa₃-Yaa₁-(G-Xaa₁-Xaa₂-G-E-Xaa₃)ₙ₂-Yaa₂-(G-Xaa₁-Xaa₂-G-E-Xaa₃)ₙ₃-Yaa₃-(G-Xaa₁-Xaa₂-G-E-Xaa₃)ₙ₃-Yaa₄-(G-Xaa₁-Xaa₂-G-E-Xaa₃)ₙ₄-.........-Yaaₙ-(G-Xaa₁-Xaa₂-G-E-Xaa₃)ₙ-Yaaₙ+₁-G-Xaa₁-Xaa₂-]N dans lequel : Yaa₁, Yaa₂, Yaa₃, Yaa₄, ........, Yaaₙ et Yaaₙ₊₁ à chaque occurrence sont choisis chacun indépendamment dans le groupe constitué par une absence, un ou plusieurs acides aminés identiques ou différents ; n2, n3, n4, ..., et n sont des nombres entiers supérieurs à 0, et deux d'entre eux ne sont pas 0 en même temps.

6. Hydrogel selon la revendication 1, **caractérisé en ce que** la séquence du collagène recombinant ne comprend aucune étiquette protéique (protein tag).

7. Hydrogel selon la revendication 1, **caractérisé en ce que** la séquence d'acides aminés du collagène recombinant est SEQ ID NO: 1.

8. Hydrogel selon la revendication 1, **caractérisé en ce que** l'anhydride est l'un parmi un anhydride insaturé cyclique ou un anhydride insaturé contenant un groupe carboxyle ou l'anhydride 4-penténoïque, l'anhydride crotonique et l'anhydride méthacrylique.

9. Hydrogel selon la revendication 1, **caractérisé en ce que** : l'anhydride est l'un parmi l'anhydride maléique, l'anhydride citraconique, l'anhydride cis-aconitique, l'anhydride 4-penténoïque, l'anhydride crotonique et l'anhydride méthacrylique ; le polysaccharide naturel est choisi parmi l'acide hyaluronique, la carboxyméthylcellulose, la méthylcellulose, l'hydroxyéthylcellulose, l'hydroxypropylcellulose, l'acide alginique, le glucane, l'agarose, l'héparine, le sulfate de chondroïtine, l'éthylène glycol chitosane, le propylène glycol chitosane, le lactate de chitosane, le carboxyméthyl chitosane ou un sel d'ammonium quaternaire de chitosane ; l'agent d'activation est le NHS et l'EDC ; le diol vicinal aminé est le 3-amino-1,2-propanediol ; et l'agent oxydant est choisi parmi un périodate et un hypochlorite ; en outre, l'agent oxydant est le périodate de sodium.

10. Procédé de préparation de l'hydrogel à formation par photodurcissement transdermique selon la revendication 1, **caractérisé en ce que** l'hydrogel est obtenu par un procédé de photodurcissement transdermique, et les étapes spécifiques sont les suivantes :
S1, une modification chimique appropriée est appliquée à un polysaccharide naturel en utilisant un anhydride pour obtenir un dérivé de polysaccharide naturel ayant des propriétés de photoréticulation ;
S2, mélange du dérivé de polysaccharide naturel ci-dessus ayant des propriétés de photoréticulation et d'un agent d'activation pour réaction, la valeur de pH de la solution réactionnelle étant de 4,65 à 5,2 ;
S3, ajout de diol vicinal aminé au système réactionnel de S2 pour une réaction d'activation de carboxyle de chaîne latérale, purification du mélange réactionnel résultant par dialyse, après quoi la solution résultante est lyophilisée pour obtenir un dérivé de polysaccharide naturel contenant une chaîne latérale diol vicinal ;
S4, dissolution du dérivé de polysaccharide naturel ci-dessus contenant une chaîne latérale diol vicinal puis réaction avec un agent oxydant, purification du mélange réactionnel résultant par dialyse,
après quoi la solution est lyophilisée pour obtenir un dérivé de polysaccharide naturel contenant une chaîne latérale aldéhyde ; et
S5, dissolution du dérivé de polysaccharide naturel ci-dessus contenant une chaîne latérale aldéhyde puis mélange de celui-ci avec le collagène recombinant et un photoinitiateur pour obtenir une solution précurseur de matrice de réparation, soumission du mélange à une photoréticulation transdermique pour obtenir l'hydrogel.

11. Procédé de préparation selon la revendication 10, **caractérisé en ce que** le degré de modification du dérivé de polysaccharide naturel ayant des propriétés de photoréticulation dans l'étape S1 est de 30 % à 60 % ; avec un rapport molaire de 1 : (1-3) : (2-5) (dérivé de polysaccharide naturel ayant des propriétés de photoréticulation : NHS : EDC).

12. Procédé de préparation selon la revendication 10, **caractérisé en ce que**, dans l'étape S2, le rapport molaire est de 1 : 2 : 3 (dérivé de polysaccharide naturel ayant des propriétés de photoréticulation : NHS : EDC).

13. Procédé de préparation selon la revendication 10, **caractérisé en ce que**, dans l'étape S3, la valeur de pH de la solution pour la réaction d'activation à laquelle un diol vicinal aminé est ajouté est de 7 à 7,7, et le rapport molaire est de (1-10) : 1 (diol vicinal aminé : dérivé de polysaccharide naturel ayant des propriétés de photoréticulation) ; le temps de dialyse dans S3 et S4 est de 20 heures à 72 heures, et la masse moléculaire retenue par dialyse est de 8 000 kDa à 14 000 kDa.

14. Procédé de préparation selon la revendication 10, **caractérisé en ce que**, dans l'étape S5, le rapport masse sur volume du dérivé de polysaccharide naturel contenant une chaîne latérale aldéhyde est de 1 % à 5 % ; le rapport masse sur volume du collagène recombinant est de 0,5 % à 2 % ; et le rapport masse sur volume de concentration finale du photoinitiateur est de 0,01 % à 0,05 %.

15. Application non thérapeutique de l'hydrogel à formation par photodurcissement transdermique doté d'une activité biologique selon la revendication 1 dans le comblement et la réparation des tissus mous.
